# FASCICULE DE BREVET EUROPEEN

(11) **EP 1 032 557 B1**
(45) Date de publication et mention de la délivrance du brevet: **10.09.2003**
(21) Numéro de dépôt: 98954537.1
(22) Date de dépôt: 09.11.1998
(51) Int. Cl.: C07C 253/30, C07C 255/50

(54) **PROCEDE DE PREPARATION DE DERIVES DE BROMOMETHYL-BIPHENYLE**
VERFAHREN ZUR HERSTELLUNG VON DERIVATEN VON BROMMETHYL-BIPHENYL VERBINDUNGEN
METHOD FOR PREPARING BROMOMETHYL-BIPHENYL DERIVATIVES

(30) Priorité: 17.11.1997 FR 9714376
(43) Date de publication de la demande: 06.09.2000
(73) Titulaire: SANOFI-SYNTHELABO, 75013 Paris (FR)
(72) Inventeur: BIARD, Michel, F-04200 Sisteron (FR); CASTRO, Bertrand, F-94270 Kremlin-Bicêtre (FR)
(74) Mandataire: Thouret-Lemaitre, Elisabeth
(86) Numéro de dépôt international: FR9802383
(87) Numéro de publication internationale: WO99025681

(56) Documents cités:
- EP-A- 0 709 369
- DATABASE WPI Section Ch, Week 9619 Derwent Publications Ltd., London, GB; Class B05, AN 96184719 XP002075733 & JP 08 059515 A (NIPPON KAYAKU KK ET AL) , 5 mars 1996
- PATENT ABSTRACTS OF JAPAN vol. 95, no. 1, 28 février 1995 & JP 06 298683 A (SANKYO CO LTD), 25 octobre 1994 cité dans la demande

## Description

La présente invention se rapporte, d'une manière générale, à la préparation de dérivés de bromométhyl-biphényle.
Plus précisément, l'invention concerne un procédé pour la préparation de dérivés de 4'-bromométhyl-biphényle de formule générale : 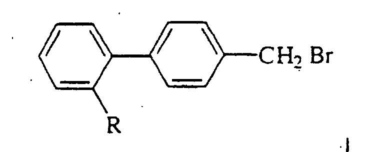 dans laquelle R représente :
- un groupement cyano
- un groupement :

   -CO₂ R₁ ou - CON R₂ R₃
dans lequel R₁, R₂, et R₃, identiques ou différents, représentent l'hydrogène ou un groupement alkyle, linéaire ou ramifié, en C₁-C₆ lequel peut être éventuellement substitué par un atome d'halogène, un groupement hydroxy, amino, alkoxy en C₁-C₄ ou par 1 à 3 groupements phényle éventuellement substitués par 1 à 3 groupements choisis parmi des atomes d'halogène et des groupements hydroxy, nitro, alkyle en C₁-C₄ et alkoxy en C₁-C₄,
- un groupement tétrazolyle de formule générale : 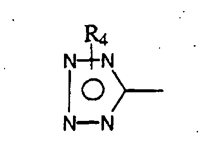
dans lequel R₄ situé en position 1 ou de préférence en position 2 du groupement tétrazolyle, représente l'hydrogène ou un groupement protecteur, en particulier un groupement alkyle, linéaire ou ramifié, en C₁-C₆ éventuellement substitué par 1 à 3 groupements phényle eux-mêmes éventuellement substitués par 1 à 3 groupements choisis parmi des atomes d'halogène et des groupements hydroxy, nitro, alkyle en C₁-C₄ et alkoxy en C₁-C₄.

En particulier, R peut représenter un groupement cyano, tétrazolyle, triphénylméthyl-tétrazolyle ou un groupement -CO₂ R₁ dans lequel R₁ représente l'hydrogène, un. groupement méthyle, éthyle ou propyle.

Parmi les composés de formule I pouvant être préparés par le procédé de l'invention, on peut citer :
le 4'-bromométhyl-2-cyanobiphényle
le 4'-bromométhyl-2-tétrazolylbiphényle
le 4'-bromométhyl-2-carboxybiphényle
le 4'-bromométhyl-2-méthoxycarbonylbiphényle
le 4'-bromométhyl-2-N-triphénylméthyltétrazolyl-biphényle

Selon un aspect préférentiel, l'invention se rapporte toutefois à la préparation du composé de formule I dans laquelle R représente le groupement cyano, c'est-à-dire le 4'-bromométhyl-2-cyanobiphényle.

Les dérivés de 4'-bromométhylbiphényle de formule I sont des composés connus ayant été décrits notamment dans les demandes de brevet EP 324377 et 553879 ou pouvant être préparés par les procédés y décrits.

Ces composés de formule I constituent des intermédiaires particulièrement utiles dans la synthèse de nombreux principes actifs de médicaments agissant notamment contre l'hypertension par un mécanisme d'inhibition de l'angiotensine II.

On a proposé récemment différentes méthodes pour la synthèse du 4'-bromométhyl-2-cyanobiphényle, ces méthodes étant basées sur une réaction radicalaire initiée chimiquement.

Par exemple, on peut citer les demandes de brevet JP 63-23868, 61-92170 et 62-98683 ainsi que les demandes de brevet EP 553879, 595150 et 709369 qui toutes décrivent, à des degrés divers, la bromation de l'o-tolylbenzonitrile, (ci-après dénommé OTBN) au moyen d'agents de bromation tels que N-bromosuccinimide (ci-après dénommée NBS), dibromodiméthylhydantoïne (ci-après dénommée DBDMH), N-bromophtalimide ou brome, en présence d'un initiateur chimique, généralement le peroxyde de benzoyle ou de t-butyle, le perbenzoate de t-butyle ou un dérivé azobis tel que le 2,2'-azobis (isobutyronitrile) (ci-après dénommé AIBN) ou le 2,2'-azobis (2,4-diméthylvaléronitrile), le solvant étant un alkane en C₅-C₇, un hydrocarbure aliphatique halogéné en C₁-C₄ tel que le dichlorométhane ou le tétrachlorure de carbone, un ester d'alkyle en C₁-C₄ de l'acide acétique tel que l'acétate d'éthyle ou encore un hydrocarbure aromatique halogéné tel que le chlorobenzène.

Toutefois, ces méthodes comportent à la fois inconvénients et désavantages, parfois suffisants pour les écarter de toute utilisation à l'échelle industrielle.

Par exemple, la mise en oeuvre de dérivés peroxydes s'avère très difficile voire dangereuse étant donné le caractère explosif de ce type de produit.

D'autre part, ces procédés font appel à des composés relativement coûteux tels que les initiateurs chimiques issus de la famille des dérivés azobis ou encore à des produits sources de pollution puisqu'ils peuvent donner naissance à des sous-produits réactionnels dont on n'a pas envisagé ou dont il est difficile de prévoir le recyclage.

En conséquence, la préparation du 4'-bromométhyl-2-cyano-biphényle par réaction radicalaire, non susceptible de pollution, à partir d'un minimum de composés, qui plus est peu coûteux et non dangereux, et selon une méthode dénuée de difficultés majeures pour sa mise en oeuvre sur le plan industriel reste d'un intérêt incontestable.

A cette fin, l'initiation photochimique d'une réaction radicalaire en remplacement d'une initiation chimique peut, en raison des avantages qu'elle procure, représenter une approche particulièrement intéressante pour offrir une solution aux problèmes posés précédemment.

Il est connu, en effet, que l'initiation chimique :
a) peut être inhibée par la présence d'impuretés dans les produits de départ comme peut l'être une réaction chimique
b) est dépendante de la température; à titre d'exemple, les initiateurs chimiques tels que peroxyde de benzoyle ou AIBN, nécessitent une certaine température d'utilisation, de préférence une température supérieure à 60°C notamment pour la bromation de l'OTBN
c) génère des produits de décomposition et, par conséquent, présente des risques de pollution de l'environnement.

Une réaction radicalaire pour la préparation du 4'-bromométhyl-2-cyano-biphényle faisant appel à une initiation photochimique a été suggérée dans les demandes de brevet JP 62-98683 et EP 709369.

Ainsi, la demande de brevet JP 62-98683 mentionne la possibilité de réaction photochimique de l'OTBN avec un agent bromant à une température de 0 à 80°C, de préférence de 10 à 40°C et dans un ester d'acide gras comme solvant, l'acétate d'éthyle étant cité plus particulièrement.

En outre, la NBS et la DBDMH y sont notées comme agents de bromation préférés et y sont exemplifiées. Si le brome y est également mentionné comme agent bromant, aucun exemple n'illustre toutefois son utilisation.

Des essais d'orientation effectués dans le cadre de l'élaboration de la présente invention ont révélé certains inconvénients inhérents à cette méthode en raison de la nature du solvant utilisé. On a remarqué en effet que des réactions parasites se produisent, impliquant le solvant à savoir l'acétate d'éthyle, ce qui provoque, une consommation accrue de l'agent de bromation, en particulier le brome.

De même, la demande de brevet EP 709369 rapporte un procédé de bromation de l'OTBN selon lequel on fait réagir ce composé avec le brome à une température de 0 à 100°C, de préférence 20 à 80°C et en présence d'un initiateur chimique, c'est-à-dire un dérivé azobis ou le peroxyde de benzoyle et dans un hydrocarbure halogéné ou un alkane en C₅-C₇ comme solvant.

On y signale également que la formation de radicaux peut être assurée par une simple photoirradiation sans l'usage d'initiateur radicalaire.

Toutefois, aucune précision quelle qu'elle soit n'est apportée notamment sur les conditions opératoires pour la mise en oeuvre de ce procédé qui n'est en aucune manière exemplifié.

Dans le cadre de la présente invention, on a tenté de réaliser cette réaction photochimique radicalaire.

On a pu ainsi remarquer que la bromation de l'OTBN par le brome avec photoirradiation est lente à des températures inférieures à 50°C dans les solvants organiques conventionnellement utilisés pour ce type de réaction. Par exemple, à 40°C dans le dichlorométhane, la réaction nécessite au moins 3 heures tandis qu'à 0°C, le temps de réaction est supérieur à 6 heures.

Etant donné que la sélectivité de cette bromation radicalaire en dérivé monobromé au détriment du dérivé dibromé correspondant augmente à mesure que la température diminue, il apparaît primordial de pouvoir opérer à des températures inférieures ou égales à 45°C.

On mentionnera également le brevet EP 336567 relatif à un procédé de bromation photochimique d'un alkylarène au moyen d'acide bromhydrique/peroxyde d'hydrogène dans un milieu biphasique formé d'une phase aqueuse et d'une phase organique et à une température de 20 à 80°C, de préférence de 60 à 70°C.

Le procédé de ce brevet peut être appliqué notamment à la bromation du carbone α d'un groupement alkyle fixé sur un cycle benzénique lui-même porteur d'un groupement désactivant comme un groupement cyano. Toutefois, la bromation de l'OTBN n'est ni décrite ni même envisagée dans ce brevet.

A la suite d'essais préliminaires effectués en cours d'élaboration de la présente invention, on a pu montrer que le procédé de ce brevet, appliqué à la bromation de l'OTBN, nécessite un temps de contact très important pour l'obtention de rendements élevés de conversion.

Or, il a maintenant été trouvé de manière inattendue qu'il est possible d'obtenir les composés de fomule I en particulier le 4'-bromométhyl-2-cyanobiphényle selon une réaction de bromation photochimique radicalaire quasi instantanée tout en évitant les inconvénients et désavantages des méthodes antérieures.

Selon l'invention, on obtient les dérivés de biphényle de formule I en faisant réagir dans un milieu biphasique et sous l'effet d'une photoirradiation, un agent de bromation choisi parmi le brome, le N-bromoacétamide, le N-bromophtalimide, le N-bromomaleimide, un N-bromosulfonamide, le N-bromosuccinimide, la 1,3-dibromo-5,5-diméthylhydantoïne et un système acide bromhydrique/bromate de métal alcalin avec un dérivé de biphényle de formule générale: 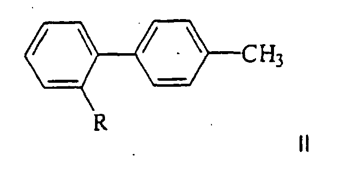 dans laquelle R a la même signification que précédemment en particulier cyano, ce qui fournit le composé désiré.

Le milieu biphasique, utilisé dans le procédé ci-dessus, est constitué d'eau et d'un ou de plusieurs solvants organiques non miscibles à l'eau choisis parmi des solvants généralement utilisés dans des réactions radicalaires et offrant peu de possibilités d'interaction avec l'agent de bromation.

De tels solvants peuvent être par exemple des hydrocarbures aliphatiques halogénés en C₁-C₄ tels que le dichlorométhane, le dichloroéthane, le chloroforme, le tétrachlorure de carbone, le tétrachloréthane ou des hydrocarbures aromatiques halogénés en C₆-C₁₀ tels que le chlorobenzène ou le dichlorobenzène.

Le dichlorométhane constitue toutefois un solvant organique préféré.

Le milieu biphasique en question comprend habituellement de 0,1 à 1 volume d'eau par volume de solvant organique et est utilisé à raison de 3 à 30 équivalents en volume par équivalent en poids de composé de formule II, en particulier d'OTBN,

Quant à l'agent de bromation, il intervient dans la réaction à raison de 0,5 à 1,4 équivalent molaire par équivalent molaire de composé de formule II, de préférence de 0,9 à 1,2 équivalent molaire, mieux encore de 0,95 à 1,1 équivalent molaire.

Au-delà de 1,2 équivalent d'agent de bromation, la tendance à la formation du dérivé dibromé correspondant s'accroît de manière sensible.

Cet agent de bromation est le brome ou un composé organique contenant du brome, choisi parmi le N-bromoacétamide, le N-bromophtalimide, le N-bromomaléimide, un N-bromosulfonamide, le N-bromosuccinimide (NBS) et la 1,3-dibromo-5,5-diméthylhydantoïne (DBDMH).

Toutefois, on retient plus particulièrement la NBS, la DBDMH ou le brome comme agents bromants préférés.

Alternativement, l'agent de bromation peut être constitué d'un système acide bromhydrique/bromate de métal alcalin, de préférence bromate de sodium.

Dans ce cas, le bromate de métal alcalin est utilisé à raison de 0,3 à 0,4 équivalent molaire par équivalent molaire de composé de formule II.

La réaction selon l'invention est habituellement conduite à une température comprise entre 0°C et 45°C. Toutefois, on préfère une température comprise entre 0°C et la température ambiante (environ de 20 à 25°C), en particulier une température comprise entre 0°C et 15°C.

Au-delà de 50 à 60°C, on remarque en fait une diminution relativement importante de la sélectivité en composé de formule I au profit du composé dibromé correspondant.

De manière à provoquer la formation de brome radicalaire nécessaire à l'initiation de la réaction de l'invention, on assure la photoirradiation du mélange réactionnel à partir d'une source lumineuse appropriée émettant des radiations de longueur d'onde inférieure à 600 nm. On peut obtenir une proportion significative de radiations efficaces à partir de lampes dont l'émission principale se situe dans la gamme de 350 à 600 nm.

Ainsi, on peut par exemple utiliser une lampe à vapeur de mercure moyenne pression émettant principalement dans cette gamme de longueurs d'onde.

Le modèle de réacteur utilisé pour la mise en oeuvre du procédé de l'invention . contribue lui aussi de manière significative à l'efficacité de l'irradiation de même que certains autres facteurs tels que le rapport du volume réactionnel à la surface de l'aire illuminée ou la distance de la source lumineuse au milieu réactionnel.

Le choix de la géométrie de ce réacteur est d'ailleurs lié à la possibilité de disposer la lampe de la manière la mieux appropriée pour optimiser l'irradiation.

Selon les cas, on peut par exemple concevoir le réacteur sous forme d'un simple tube en U coudé ou d'un tube spiralé de telle manière que le système d'éclairage puisse être positionné à proximité immédiate de ce réacteur.

Préférentiellement, on choisit un type de réacteur offrant la possibilité de disposer la lampe de manière coaxiale par exemple un réacteur sous forme de serpentin ou de structure tubulaire annulaire.

De manière à réduire encore davantage les pertes en radiations lumineuses, on peut adjoindre également des réflecteurs.

De surcroît, il est impératif de tenir compte de facteurs capables d'affecter l'efficacité du flux lumineux par unité de surface à irradier et ce, en faisant appel à des réacteurs dont le modèle est adapté de la manière la plus appropriée.

En conséquence, le dispositif destiné à la mise en oeuvre du procédé de l'invention, sera choisi ou modifié de manière à minimiser ou éliminer les volumes de mélange réactionnel qui ne reçoivent pas directement de radiations lumineuses. Pour cette raison, le choix du réacteur se portera de préférence sur les modèles de type annulaire ou en forme de serpentin de manière à pouvoir disposer la lampe de façon coaxiale.

On prévoira également un système capable de produire une agitation ou une turbulence efficace du mélange réactionnel de manière à augmenter les possibilités de passage de ce mélange au travers d'une zone illuminée du réacteur.

Le procédé de l'invention ainsi décrit se déroule sur une période d'environ 1 à 2 heures selon la température réactionnelle en application et la durée de la phase d'introduction des réactifs, dans la plupart des cas sur une période d'environ 1,5 heure.

Ainsi, à des températures de l'ordre de 35°C à 45°C, la réaction est terminée en 1 heure équivalant à la durée d'introduction, dans le milieu réactionnel, du ou des réactifs à savoir l'agent de bromation ou un mélange d'agent de bromation / composé de formule II.

Par contre, à des températures variant d'environ 0°C à environ 15°C, la réaction est achevée en environ 1,5 heure si la durée d'introduction des réactifs est également d'environ 1 heure.

On observe, en conséquence, que la réaction selon l'invention est quasi instantanée à des températures de l'ordre de 35°C à 45°C notamment à la température de reflux du dichlorométhane. En outre, à des températures d'environ 0°C à environ 15°C, la durée de la réaction reste tout à fait compatible avec un développement industriel.

A titre comparatif, on a effectué la photobromation de l'OTBN par le brome à la température de 40°C soit dans le dichlorométhane seul soit dans un milieu biphasique selon l'invention c'est-à-dire constitué d'eau et de dichlorométhane, en vue de former le 4'-bromométhyl-2-cyanobiphényle ci-après dénommé Composé A.

Dans les deux cas, les mêmes conditions réactionnelles ont été respectées et l'introduction du brome dans le milieu réactionnel contenant l'OTBN nécessite environ 1 heure.

On a obtenu les résultats suivants exprimés en % d'OTBN, de Composé A et de
4'-dibromométhyl-2-cyanobiphényle (ci-après dénommé "composé dibromé") relevés dans le milieu réactionnel :

### a) dans le dichlorométhane seul

| Temps (min) | OTBN restant (%) | Composé A formé (%) | Composé dibromé formé (%) |
|---|---|---|---|
| 15 | 99,7 | 0,3 | 0 |
| 30 | 73,4 | 26,1 | 0,5 |
| 60 | 46,5 | 53 | 0,5 |
| 120 | 13,6 | 80,6 | 5,8 |
| 180 | 9,5 | 82,9 | 7,6 |

### b) dans le milieu biphasique selon l'invention

| Temps (min) | OTBN restant (%) | Composé A formé (%) | Composé dibromé formé (%) |
|---|---|---|---|
| 15 | 60,10 | 39,90 | 0 |
| 30 | 31,78 | 66,02 | 2,2 |
| 57 | 8,44 | 83,92 | 7,64 |
| 120 | 8,40 | 84,18 | 7,42 |

Ces résultats montrent :
- que la réaction selon l'invention est instantanée à la température utilisée puisque le rendement final est pratiquement obtenu dès la fin de l'introduction des réactifs
- que le procédé de l'état de la technique nécessite au moins 3 heures pour obtenir un rendement en Composé A avoisinant le rendement obtenu après 1 heure par mise en oeuvre du procédé selon l'invention.

D'autres essais comparatifs de photobromation de l'OTBN ont été pratiqués à 10°C dans un milieu biphasique eau/dichlorométhane avec un système acide bromhydrique/bromate de sodium comme agent de bromation comparativement à un système acide bromhydrique/peroxyde d'hydrogène.

Les résultats ont montré que la réaction avec le système acide bromhydrique/peroxyde d'hydrogène ne conduit qu'à une conversion de 20% d'OTBN en Composé A après 1,5 heure. Par contre, dans les mêmes conditions, on a enregistré également après 1,5 heure un taux de conversion de 85% d'OTBN en Composé A. En outre, la bromation au départ d'acide bromhydrique/bromate de sodium s'est révélée quasi instantanée.

Par ailleurs, les résultats ont montré qu'en fin de réaction la sélectivité en Composé A est meilleure avec le système acide bromhydrique/bromate de sodium qu'avec le système acide bromhydrique/peroxyde d'hydrogène.

Enfin, les dérivés monobromés de formule I, obtenus selon le procédé de l'invention ainsi décrit, peuvent être ultérieurement séparés du milieu réactionnel selon des méthodes classiques par exemple par décantation de la phase organique, élimination du solvant et éventuellement recristallisation dans un milieu approprié ou encore par des méthodes chromatographiques.

Le procédé de l'invention peut être mis en oeuvre de différentes manières en faisant appel, selon les cas, à des techniques de type discontinu ou continu.

Par exemple, on peut envisager la préparation des composés de formule I selon l'invention, à partir de mises en oeuvre en phase discontinue impliquant :
1) ou bien le chargement total, dans le réacteur, des divers constituants du milieu biphasique ainsi que des réactifs à savoir le substrat de formule II et l'agent de bromation, puis l'irradiation du milieu réactionnel
2) ou bien le chargement des divers constituants du milieu biphasique puis, dans ce milieu maintenu sous irradiation, l'introduction d'un mélange des réactifs, éventuellement en solution ou suspension dans le solvant organique,
3) ou au contraire l'introduction par coulée de l'agent de bromation éventuellement en solution ou suspension dans le solvant organique et ce, dans le milieu réactionnel maintenu sous irradiation, milieu constitué du mélange biphasique contenant le substrat de formule II. Dans le cas d'un agent de bromation formé d'acide bromhydrique bromate de métal alcalin, on peut aussi envisager l'introduction par coulée de l'acide bromhydrique dans un milieu réactionnel sous irradiation lumineuse, milieu constitué du mélange biphasique, du bromate de métal alcalin et du substrat de formule II.

Pour la mise en oeuvre du procédé de l'invention selon une méthode discontinue, on peut faire appel à un dispositif comportant un réacteur de type classique muni d'une double enveloppe de chauffage où de refroidissement et conçue de manière à permettre de préférence l'immersion de la source lumineuse dans le milieu réactionnel.

Dans le cadre de la mise au point du procédé de l'invention, on a pu observer une augmentation de la sélectivité en dérivé monobromé de formule I lorsque la température réactionnelle décroit mais aussi lorsque les réactifs sont mélangés en totalité avant introduction dans le réacteur.

Toutefois, la mise en oeuvre du procédé de l'invention par introduction du mélange des réactifs tel que prévu dans les techniques discontinues 2) et 3) décrites ci-dessus nécessite un contrôle quasi parfait de la quantité d'initiateur photochimique pour maîtriser la cinétique de la réaction. Comme la puissance des lampes responsables de cette initiation photochimique n'est pas réglable, il s'avère nécessaire, par conséquent, d'irradier de petits volumes de ce milieu dont on maîtrise plus aisément la température.

En raison de ces contraintes, on peut envisager favorablement le déroulement du procédé de l'invention en phase continue.

Ainsi, on peut faire appel, pour la réaction de bromation, à des mises en oeuvre en phase continue basées sur une circulation des réactifs constitués, de l'agent de bromation et du dérivé de biphényle de formule II, dans un réacteur tubulaire à écoulement piston ("plug-flow reactor"), c'est-à-dire à écoulement tel que chaque élément de fluide traverse le réacteur sans mélange avec le précédent ou le suivant, ce réacteur étant photoirradié et maintenu à une température comprise entre 0°C et 45°C, par exemple à une température comprise entre 0°C et la température ambiante, de préférence à une température comprise entre 0°C et 15°C.

A cette fin, on peut utiliser avantageusement un dispositif approprié permettant, par exemple, l'introduction à débit contrôlé et dans un milieu biphasique, d'une solution ou suspension de substrat de formule II et d'agent de bromation éventuellement dans le solvant organique choisi, le débit étant régulé par un ensemble composé d'une pompe doseuse et d'un régulateur de débit couplé à une balance. Ce dispositif est en outre complété par un réacteur comportant une entrée, pour l'introduction des divers constituants du milieu réactionnel et une sortie pour recueillir les produits issus de la réaction. Ce réacteur, destiné à recevoir le milieu biphasique, est constitué d'un tube par exemple en verre ou en quartz, de préférence sous forme de spirale, immergé dans un fluide de chauffage ou de refroidissement, par exemple un bain d'eau glycolée ou non, dont la température est contrôlée par un cryothermostat.

Dans un tel dispositif, lorsqu'il comporte un réacteur spiralé, la lampe nécessaire à la photoirradiation du milieu réactionnel, par exemple une lampe à vapeur de mercure, peut être positionnée à l'extérieur ou, de préférence, à l'intérieur du volume tubulaire formé par ce réacteur mais cependant à proximité immédiate de ce dernier.

Certains agents de bromation, utilisés dans le procédé de l'invention, peuvent donner naissance à des sous-produits solubles dans l'eau. Tel est notamment le cas de la DBDMH qui fournit la 5,5-diméthylhydantoïne (ci-après dénommée DMH) hydrosoluble mais qui toutefois cristallise dans le milieu si le taux de conversion du composé de formule Il en composé monobromé de formule I est supérieur à 80%.

Cette cristallisation entraîne par conséquent des phénomènes de colmatage du réacteur tubulaire qui pertubent l'écoulement piston du milieu réactionnel.

Par conséquent, on peut envisager de compléter avantageusement le procédé de l'invention par une opération d'extraction continue des sous-produits hydrosolubles issus de la réaction de bromation, leur évacuation du milieu réactionnel et, si nécessaire, leur récupération.

En conséquence et selon une mise en oeuvre préférée du procédé de l'invention on effectue, simultanément et en phases continues, la réaction de bromation et l'extraction des sous-produits hydrosolubles issus de cette réaction, sous-produits que l'on évacue ensuite du milieu réactionnel.

A cette fin, on utilise avantageusement un réacteur tubulaire à écoulement piston constitué d'une colonne d'extraction liquide/liquide.

Ces opérations peuvent être menées selon une technique impliquant d'une part la réaction de bromation en phase continue par circulation du mélange réactionnel formé par le solvant organique et les réactifs dans un réacteur tubulaire à écoulement piston constitué d'une colonne d'extraction liquide/liquide photoirradiée et maintenue à une température comprise entre 0°C et 45°C, par exemple à une température comprise entre 0°C et la température ambiante préférentiellement à une température comprise entre 0°C et 15°C et d'autre part l'extraction simultanée et continue des sous-produits hydrosolubles opérée par l'eau du milieu biphasique circulant à contre-courant dudit mélange réactionnel dans la colonne ainsi que l'évacuation des sous-produits hydrosolubles en question du milieu réactionnel.

Cette méthode permet notamment d'accroître la sélectivité de la réaction de bromation et d'en améliorer le contrôle thermique par élimination, si nécessaire, de la chaleur dégagée par la lampe et par la réaction.

Un autre objet de l'invention se rapporte, en conséquence, à un dispositif de photobromation pour la mise en oeuvre du procédé de l'invention en phase continue, comportant un réacteur tubulaire à écoulement piston constitué d'une colonne d'extraction liquide/liquide photoirradiée et maintenue à une température comprise entre 0°C et 45°C, par exemple à une température comprise entre 0°C et la température ambiante, de préférence à une température comprise entre 0°C et 15°C.

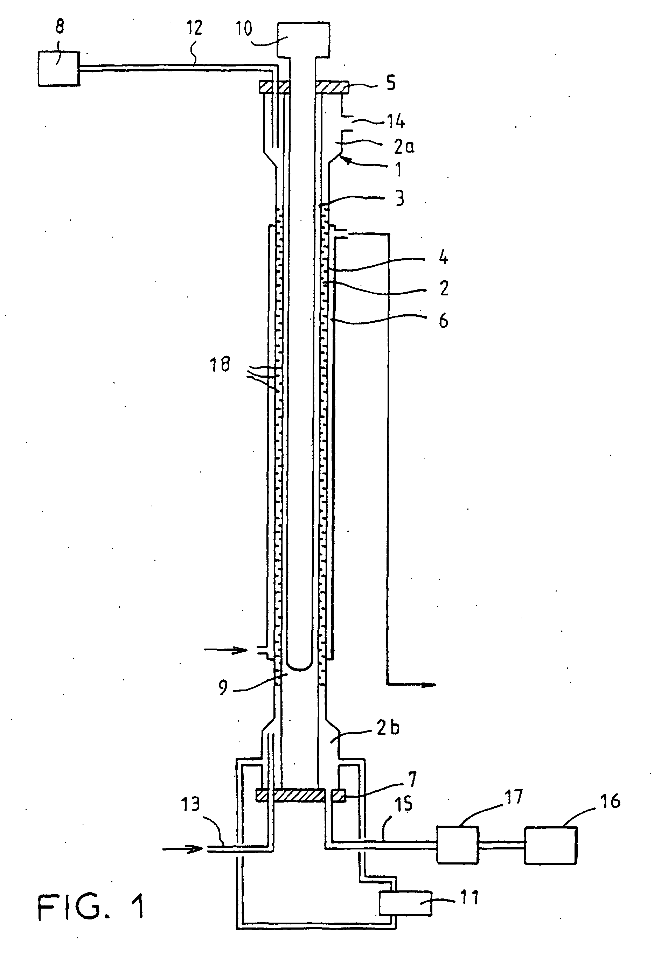

D'autres caractéristiques et avantages apparaîtront au cours de la description suivante d'un mode de réalisation du dispositif de l'invention, donné à titre d'exemple non limitatif, en regard au dessin annexé sur lequel la Figure représente une vue schématique d'un tel dispositif de photobromation continue.

Le dispositif décrit est particulièrement adapté à la mise en oeuvre d'un procédé utilisant, à titre de solvants organiques non miscibles à l'eau, des hydrocarbures aliphatiques ou aromatiques halogénés.

En référence à cette Figure, le dispositif comporte un réacteur (1) formant colonne d'extraction liquide/liquide, par exemple en verre ou en quartz.

Ce réacteur est constitué d'une enceinte annulaire (2), à axe vertical, délimitée par une paroi interne (3) et une paroi externe (4) espacées. A ses parties supérieure respectivement inférieure, l'enceinte (2) comporte deux décanteurs (2a) et (2b) ou zone de repos permettant la séparation des deux phases. Deux disques (7) et (5) ferment respectivement les extrémités inférieure et supérieure de l'enceinte (2). La paroi externe (4) est dans sa partie centrale au contact d'un échangeur de chaleur (6). Selon un mode de réalisation préféré de l'invention, l'enceinte annulaire (2) est munie dans sa partie centrale extérieure d'une double enveloppe dans laquelle circule un liquide de refroidissement ou de chauffage, par exemple une solution aqueuse glycolée.

La paroi interne (3), et les disques (7) et (5) délimitent une enceinte intérieure (9) dans laquelle est disposée une lampe photochimique (10), par exemple une lampe à vapeur de mercure, munie d'une double enveloppe (non représentée) dans laquelle circule un liquide de refroidissement, par exemple de l'eau désionisée. Alternativement, la lampe photochimique (10) est dépourvue d'une telle double enveloppe. Dans ce cas, le refroidissement de cette lampe peut être assuré par une circulation de liquide prévu à cet effet dans l'enceinte (9).

La lampe (10) traverse le disque (5) auquel elle est fixée.

Le réacteur comprend également un conduit d'alimentation (12) pour le chargement des réactifs et du solvant organique situé en tête de colonne ainsi qu'un conduit d'alimentation en eau (13), en pied de colonne. Le conduit d'alimentation en eau (13) traverse le disque (7) et débouche dans le décanteur inférieur (2b) de l'enceinte (2) au-dessous du niveau de la lampe (10). Le conduit d'alimentation (12) traverse le disque (5) et débouche dans le décanteur supérieur (2a) de l'enceinte (2).

Le réacteur comprend en outre une sortie d'évacuation de la solution aqueuse (14) pour l'évacuation des sous-produits hydrosolubles. Cette sortie est située en tête de colonne et consiste par exemple en un conduit d'évacuation traversant la paroi externe (4) de l'enceinte (2) dans le décanteur supérieur (2a). Le disque (7) est par ailleurs traversé par un conduit d'évacuation (15) des produits de la réaction. Les faces en regard des parois interne (3) et externe (4) de l'enceinte (2) comprennent de préférence des parties en saillie constituées par exemple de demi-disques imbriqués disposés le long de la partie centrale de l'enceinte (2), formant des chicanes (18).

Ces demi-disques sont constitués d'une matière inerte, par exemple de matière plastique appropriée telle que du polytétrafluoroéthylène. Alternativement, le garnissage en forme de chicanes 'est composé de couronnes ou disques supportés par des tiges (non représentées) fixées sur les disques (5) et (7), l'espace entre ces couronnes ou disques étant réglable par des entretoises de hauteur définie.

Au surplus, le dispositif de photobromation peut être équipé en amont du conduit d'alimentation (12) d'un réservoir (8) destiné au stockage des réactifs et du solvant organique et, en aval du conduit d'évacuation (15) d'un second réservoir (16) pour le stockage, après soutirage, des produits bromés formés en cours de réaction.

Avantageusement, ce dispositif comprend une garde hydraulique (17) située immédiatement en sortie de colonne, en aval du conduit d'évacuation (15) et montée en série avec le réservoir (16). Cette garde hydraulique est destinée à maintenir la phase organique de manière continue dans l'enceinte (2) en vue de réaliser la photobromation avec écoulement piston, l'eau constituant la phase dispersée.

Cette dispersion, obtenue grâce aux chicanes, est cependant améliorée par la pulsation du mélange réactionnel provoquée par une pompe doseuse (11) par exemple une pompe à membrane, reliée au décanteur inférieur (2b) de l'enceinte (2).

Lors d'une utilisation de ce dispositif, l'enceinte (2) est préalablement remplie du solvant organique. La lampe (10) est allumée et son circuit de refroidissement est mis en route.

Lorsqu'elle est présente, la pompe doseuse (11) est mise en service de façon à assurer la pulsation du mélange réactionnel au sein de l'enceinte (2).

L'enceinte (2) est portée ensuite à la température souhaitée au moyen du système de chauffage ou de refroidissement correspondant.

On procède alors à l'alimentation en continu de la colonne en eau d'une part et en solution organique des réactifs d'autre part.

L'utilisation du dispositif de bromation et d'extraction simultanées ainsi conçu, pour la mise en oeuvre du procédé de l'invention en phase continue, a permis d'obtenir les dérivés monobromés de formule I avec une excellente sélectivité et un taux de conversion du composé de formule II en composé désiré est particulièrement élevé.

Par exemple, le taux de conversion de l'OTBN en 4'-bromométhyl-2-cyanobiphényle est supérieur ou égal à 90% et la sélectivité en composé désiré comprise entre 85 et 90% tandis que le taux en composé dibromé correspondant est inférieur à 10%, en général de l'ordre de 5 à 7%.

Parmi les sous-produits issus de la réaction, ceux provenant de la décomposition de l'agent de bromation peuvent être avantageusement récupérés, retransformés et recyclés par exemple dans le procédé de l'invention.

Tel est le cas notamment de la DMH, produit hydrosoluble issu de la décomposition de la DBDMH. Ce produit, après retransformation en DBDMH, par exemple par traitement au moyen de brome en milieu basique, peut être recyclé notamment dans une mise en oeuvre continue du procédé de l'invention.

Par exemple, à 10°C, un tel traitement, en présence d'hydroxyde de sodium ou de carbonate de sodium, fournit 91 à 92% de DBDMH avec une pureté supérieure à 99%.

Cette possibilité de récupération et de recyclage de sous-produits réactionnels après retraitement ajoute à l'intérêt représenté par la mise en oeuvre du procédé de l'invention en phase continue décrite précédemment lorsqu'elle fait appel à une colonne d'extraction liquide/liquide comme réacteur de bromation à écoulement piston.

Les Exemples non limitatifs suivants illustrent l'invention.

Dans ces Exemples, les contrôles en cours de réaction ont été pratiqués par chromatographie liquide haute performance, ci-après CLHP, sur le milieu réactionnel dilué dans l'acétonitrile.

En outre, on y a désigné par "composé dibromé" le 4'-dibromométhyl-2-cyanobiphényle également produit en cours de réaction de bromation.

### EXEMPLE 1

### 4'-Bromométhyl-2-cyanobiphényle

Dans un réacteur en verre de 2 litres muni d'une double enveloppe reliée à un cryothermostat permettant de régler la température interne, d'un agitateur de type turbine et d'une lampe à vapeur de mercure moyenne pression (puissance : 150 watts) située dans un tube plongeant refroidi par une circulation d'eau dans une double enveloppe, on introduit 200 g (1,036 mole) d'OTBN, 1000 ml de dichlorométhane et 500 ml d'eau puis on met l'ensemble sous agitation.

On allume la lampe, on chauffe le milieu réactionnel avec celle-ci jusqu'à ébullition puis, tout en régulant le débit d'alimentation, on introduit en 1 heure, une suspension de 162,2 g (0,567 mole) de DBDMH dans 200 mi de dichlorométhane.

On maintient le milieu à reflux pendant l'introduction puis ensuite durant 1 heure supplémentaire. On refroidit le mélange réactionnel entre 20 et 25°C, on arrête l'agitation et on laisse décanter pendant 30 min.

On élimine la phase aqueuse supérieure et on effecute 2 lavages de la phase organique au moyen de 2000 ml d'eau.

Un contrôle par CLHP fourni les résultats suivants :

| OTBN | composé désiré | composé dibromé |
|---|---|---|
| 3,5% | 86% | 10,5% |

On isole alors le composé souhaité après élimination partielle du dichlorométhane, rajout de 250 ml d'éther de méthyle et de tert-butyle, élimination du dichlorométhane en maintenant le volume constant par ajout successifs de fractions de 250 ml d'éther de méthyle et de tert-butyle (% final de dichlorométhane < 2,5), refroidissement à 20°C, filtration, essorage, lavage avec 150 ml puis 70 ml d'éther de méthyle et de tert-butyle et séchage en étuve à 50°C sous vide.

De cette manière, on obtient 224,5g de 4'-bromométhyl-2-cyanobiphényle sous forme d'une poudre beige.

Rendement : 79,6%

### EXEMPLE 2

### 4'-Bromométhyl-2-cyanobiphényle

Dans un réacteur en verre équipé comme décrit à l'Exemple 1, on introduit 150 g (0,776mole) d'OTBN, 700 ml de dichlorométhane et 300 ml d'eau puis on met l'ensemble sous agitation à 300 tours/min. On allume la lampe à vapeur de mercure puis, tout en régulant le débit d'alimentation, on introduit en environ 1 heure, une solution de 124 g (0,776 mole) de brome dans 200 ml de dichlorométhane. On maintient le milieu à reflux durant la durée totale d'introduction.

Un contrôle par CLHP effectué après 57 min montre que le milieu contient :

| OTBN | composé désiré | composé dibromé |
|---|---|---|
| 8,44% | 83,92% | 7,64% |

On maintient le milieu à reflux durant 1 heure supplémentaire puis on refroidit entre 20 et 25°C. On arrête l'agitation et on laisse décanter pendant 30 min.

On élimine la phase aqueuse supérieure et on effectue 2 lavages de la phase organique au moyen d'eau.

Un nouveau contrôle par CLHP a fourni les résultats suivants après 2 heures de réaction :

| OTBN | composé désiré | composé dibromé |
|---|---|---|
| 8,4% | 84,18% | 7,42% |

### EXEMPLE 3

### 4'-Bromométhyl-2-cyanoblphényle

Dans un réacteur en verre équipé comme à l'Exemple 1, on introduit 200 g (1,036 mole) d'OTBN, 900 ml de dichlorométhane et 400 ml d'eau puis on met l'ensemble sous agitation à 380 tours/min.

On refroidit le milieu à une température comprise entre 0°C et 5°C, puis on allume la lampe à vapeur de mercure tout en maintenant le milieu réactionnel à la même température. Tout en régulant le débit d'alimentation, on introduit ensuite, en environ 1 heure, une solution de 165,4 g (1,03 mole) de brome dans 300 ml de dichlorométhane.

On maintient le milieu à une température comprise entre 0°C et 5°C durant la durée totale d'introduction.

En fin d'introduction, c'est-à-dire après 58 min, un contrôle par CLHP du milieu réactionnel coloré montre qu'il contient :

| OTBN | composé désiré | composé dibromé |
|---|---|---|
| 34,05% | 64,66% | 1,29% |

On continue l'agitation, ce qui fournit 30 min après la fin de l'introduction, un milieu orangé contenant :

| OTBN | composé désiré | composé dibromé |
|---|---|---|
| 14,16% | 82,60% | 3,24% |

60 min après la fin de l'introduction, ce même milieu devenu jaune contient :

| OTBN | composé désiré | composé dibromé |
|---|---|---|
| 6,53% | 88,42% | 5,05% |

### EXEMPLE 4

### 4'-Bromométhyl-2-cyanobiphényle

Dans un réacteur de 2 litres en verre équipé comme décrit à l'Exemple 1, on introduit 250 g (1,29 mole) d'OTBN, 1000 ml de dichlorométhane et 500 ml d'eau puis on met l'ensemble sous agitation à 300 tours/min. On refroidit le milieu à 10°C par circulation d'eau glycolée dans la double enveloppe puis on ajoute 64,9 g (0,43 mole) de bromate de sodium.

On allume la lampe à vapeur de mercure puis on débute l'introduction de 222 g d'acide bromhydrique à 47% (correspondant à 104,4 g d'acide à 100% soit 1,29 mole) au moyen d'une pompe doseuse et d'un régulateur de débit (débit : 222 g/h).

En fin d'introduction de l'acide bromhydrique c'est-à-dire après 60 min le milieu coloré en orange renferme :

| OTBN | composé désiré | composé dibromé |
|---|---|---|
| 32,27% | 66,27% | 1,46% |

On maintient ce milieu à 10°C sous agitation durant 60 min supplémentaires et on obtient ainsi un mélange contenant :

| OTBN | composé désiré | composé dibromé |
|---|---|---|
| 5,62% | 88,02% | 6,36% |

### EXEMPLE 5

### 4'-Bromométhvl-2-cvanobiDhénvle

### a) Préparation

Dans un réservoir, on charge 100 kg d' OTBN et 81,2 kg (1,1 équivalent) de DBDMH puis on ajoute 2000 I de dichlorométhane.
On met l'ensemble sous agitation à 60 à 80 tours/min et on maintient celle-ci à 20°C à 25°C jusqu'à dissolution complète.

### b) Photobromation

On utilise pour cette opération un dispositif comprenant un réacteur tubulaire annulaire à écoulement piston sous forme d'une colonne d'extraction liquide/liquide comme illustré à la Figure en annexe.

On remplit cette colonne de dichlorométhane, on règle la puissance de la lampe à 8 kw, on la pressurise à 0,3 bar puis on met en service son circuit spécifique de refroidissement à l'eau désionisée.

On allume la lampe et on met en service le système de pulsation du mélange réactionnel dans la colonne en règlant la fréquence de pulsation à 30% (=15Hz) et de la course à 20%.

On met en service la circulation d'eau glycolée dans la double enveloppe de la colonne pulsée et on règle les débits d'eau glycolée jusqu'à obtenir une température de 10°C dans le milieu dichlorométhane.

On débute alors l'alimentation continue de la colonne pulsée d'abord en eau désionisée à raison de 300 kg/h ensuite en solution OTBN/DBDMH dans le dichlorométhane préparée au paragraphe a) ci-dessus, à raison de 600 kg/h.

On maintient ces conditions opératoires jusqu'à épuisement de la solution des réactifs dans le dichlorométhane puis on rince le réservoir ayant contenu cette solution, la ligne de transfert jusqu'à la colonne ainsi que cette dernière avec 200 I de dichlorométhane.

Une analyse par CLHP montre que le mélange en fin de réaction contient :

| OTBN | composé désiré | composé dibromé |
|---|---|---|
| 5,7% | 87,5% | 6,8% |

### c) Isolement

On isole alors le composé désiré par lavage de la solution des produits photobromés dans le dichlorométhane par une solution aqueuse diluée de bisulfite de sodium, récupération de la phase organique par décantation, préconcentration de cette phase organique, élimination du dichlorométhane par ajouts successifs d'éther de méthyle et de tert-butyle, cristallisation de la suspension à 0°C à 3°C, filtration, essorage de la suspension, lavage du produit obtenu par de l'éther de méthyle et de tert-butyle et sèchage à 40 à 45°C.

De cette manière, on obtient le 4'-bromométhyl-2-cyanobiphényle pur avec un rendement de 78% en poids par rapport à l'OTBN.

## Revendications

1. Procédé de préparation de dérivés de 4'-bromométhyl-biphényle de formule générale : 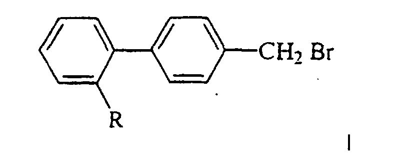 dans laquelle R représente :
• un groupement cyano
• un groupement :
- CO₂R₁ ou - CON R₂ R₃
dans lequel R_{1,} R₂ et R₃, identiques ou différents, représentent l'hydrogène ou un groupement alkyle, linéaire ou ramifié, en C₁-C₆ lequel peut être éventuellement substitué par un atome d'halogène, un groupement hydroxy, amino, alkoxy en C₁-C₄ ou par 1 à 3 groupements phényle éventuellement substitués par 1 à 3 groupements choisis parmi des atomes d'halogène et des groupements hydroxy, nitro, alkyle en C₁-C₄ et alkoxy en C₁-C₄,
• un groupement tétrazolyle de formule générale : 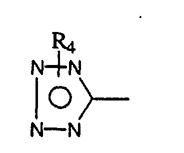
dans lequel R₄ situé en position 1 ou 2 du groupement tétrazolyle, représente l'hydrogène ou un groupement protecteur, **caractérisé en ce que** l'on fait réagir dans un milieu biphasique et sous l'effet d'une photoirradiation, un agent de bromation choisi parmi le brome, le N-bromoacétamide, le N-bromophtalimide, le N-bromomaléimide, un N-bromosulfonamide, le N-bromosuccinimide, la 1,3-dibromo-5,5-diméthylhydantoïne et un système acide bromhydrique/bromate de métal alcalin avec un dérivé de biphényle de formule générale : 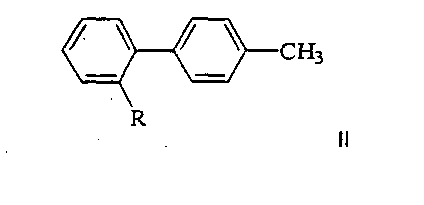 dans laquelle R a la même signification que précédemment, ce qui fournit le composé désiré.

2. Procédé selon la Revendication 1, **caractérisé en ce que** R représente un groupement tétrazolyle dans lequel R₄ représente un groupement alkyle, linéaire ou ramifié, en C₁-C₆ éventuellement substitué par 1 à 3 groupements phényle eux-mêmes éventuellement substitués par 1 à 3 groupements choisis parmi des atomes d'halogène et des groupements hydroxy, nitro, alkyle en C₁-C₄ et alkoxy en C₁-C₄.

3. Procédé selon la Revendication 1, **caractérisé en ce que** R représente un groupement cyano, tétrazolyle, triphénylméthyl-tétrazolyle ou un groupement - CO₂ R₁ dans lequel R₁ représente l'hydrogène, un groupement méthyle, éthyle ou propyle.

4. Procédé selon une des Revendications 1 ou 3, **caractérisé en ce que** R représente le groupement cyano.

5. Procédé selon une des Revendications 1 à 4, **caractérisé en ce que** le milieu biphasique est constitué d'eau et d'un ou de plusieurs solvants organiques non miscibles à l'eau.

6. Procédé selon la Revendication 5, **caractérisé en ce que** le solvant organique est un hydrocarbure aliphatique halogéné ou un hydrocarbure aromatique halogéné.

7. Procédé selon la Revendication 5 ou 6; **caractérisé en ce que** le solvant organique est le dichlorométhane.

8. Procédé selon une des Revendications 1 à 7, **caractérisé en ce que** le milieu biphasique comprend de 0,1 à 1 volume d'eau par volume de solvant organique.

9. Procédé selon une des Revendications 1 à 8, **caractérisé en ce que** le milieu biphasique est utilisé à raison de 3 à 30 équivalents en volume par équivalent en poids de dérivé de biphényle de formule II.

10. Procédé selon une des Revendications 1 à 9, **caractérisé en ce que** l'agent de bromation est le brome, le N-bromosuccinimide, la 1,3-dibromo-5,5-diméthylhydantoïne ou un système acide bromhydrique/bromate de métal alcalin.

11. Procédé selon une des Revendications 1 à 10, **caractérisé en ce que** l'agent de bromation est utilisé à raison de 0,5 à 1,4 équivalent molaire par équivalent molaire de dérivé de biphényle de formule II.

12. Procédé selon la Revendication 11, **caractérisé en ce que** l'agent de bromation est utilisé à raison de 0,95 à 1,1 équivalent molaire par équivalent molaire de dérivé de biphényle de formule II.

13. Procédé selon une des Revendications 11 ou 12, **caractérisé en ce que** le bromate de métal alcalin est utilisé à raison de 0,3 à 0,4 équivalent molaire par équivalent molaire de dérivé de biphényle de formule II.

14. Procédé selon une des Revendications 1 à 13, **caractérisé en ce que** la réaction est conduite à une température comprise entre 0°C et 45°C.

15. Procédé selon la Revendication 14, **caractérisé en ce que** la réaction est conduite à une température comprise entre 0°C et la température ambiante.

16. Procédé selon la Revendication 14 ou 15, **caractérisé en ce que** la réaction est conduite à une température comprise entre 0°C et 15°C.

17. Procédé selon une des revendications 1 à 16, **caractérisé en ce que** la photoirradiation est obtenue à partir d'une source lumineuse émettant des radiations de 350 à 600nm.

18. Procédé selon une des Revendications 1 à 14 et 17, **caractérisé en ce que** la réaction de bromation se déroule en phase continue et comprend la circulation en continu des réactifs constitués de l'agent de bromation et du dérivé de biphényle de formule II dans un réacteur tubulaire à écoulement piston, réacteur photoirradié et maintenu à une température comprise entre 0°C et 45°C.

19. Procédé selon la Revendication 18, **caractérisé en ce que** l'on effectue simultanément et en phases continues, la réaction de bromation et l'extraction des sous-produits hydrosolubles que l'on évacue ensuite du milieu réactionnel.

20. Procédé selon la Revendication 19, **caractérisé en ce que** la réaction, l'extraction et l'évacuation sont réalisées dans un réacteur à écoulement piston constitué d'une colonne d'extraction liquide/liquide.

21. Procédé selon une des Revendications 18 à 20, **caractérisé en ce qu'**il comprend, d'une part, la réaction de bromation en phase continue par circulation du mélange formé par. le solvant organique et les réactifs, dans un réacteur tubulaire à écoulement piston constitué d'une colonne d'extraction liquide/liquide photoirradiée et maintenue à une température comprise entre 0°C et 45°C et, d'autre part, l'extraction simultanée et continue des sous-produits hydrosolubles opérée par l'eau du milieu biphasique circulant à contre-courant dudit mélange dans la colonne, suivie de l'évacuation desdits sous-produits hydrosolubles du milieu réactionnel.

22. Procédé selon une des Revendications 18 à 21, **caractérisé en ce que** la température est comprise entre 0°C et la température ambiante.

23. Procédé selon une des Revendications 18 à 22, **caractérisé en ce que** la température est comprise entre 0°C et 15°C.

24. Dispositif de photobromation pour la mise en oeuvre du procédé en phase continue selon une des Revendications 18 à 23, **caractérisé en ce qu'**il comporte un réacteur tubulaire à écoulement piston constitué d'une colonne d'extraction liquide/liquide photoirradiée et maintenue à une température comprise en 0°C et 45°C.

25. Dispositif selon la Revendication 24, comportant (i) un réacteur (1) constitué d'une enceinte annulaire (2) à axe vertical, délimitée par une paroi interne (3) et une paroi externe (4) espacées, et fermée par un disque (7) situé au niveau du décanteur inférieur (2b) de l'enceinte (2) et par un disque (5) situé au niveau du décanteur supérieur (2a) de l'enceinte (2), (ii) une lampe photochimique (10) munie d'une double enveloppe de refroidissement, disposée à l'intérieur de l'enceinte (9) délimitée par la paroi interne (3) de l'enceinte (2) et par le disque (5), traversant ledit disque (5) dans le sens de l'épaisseur, (iii) un conduit d'alimentation (12) pour le chargement des réactifs et du solvant organique, situé en tête de colonne traversant le disque (5) et débouchant sur le décanteur supérieur (2a) de l'enceinte (2), (iv) un conduit d'alimentation en eau (13) situé en pied de colonne traversant le disque (7) et débouchant dans le décanteur inférieur (2b) de l'enceinte (2), (v) un conduit d'évacuation de la solution aqueuse (14) situé en tête de colonne traversant la paroi externe (4) de l'enceinte (2) au niveau du décanteur supérieur (2a) et (vi) un conduit d'évacuation (15) des produits de la réaction située en pied de colonne, traversant le disque (7), ladite enceinte annulaire (2) étant munie dans sa partie centrale d'un échangeur de chaleur (6) disposé au contact de sa paroi externe (4), les parois interne (3) et externe (4) de l'enceinte (2) étant munies dans leur partie centrale de chicanes (18).

26. Dispositif selon la Revendication 24 ou 25, **caractérisé en ce qu'**il est équipé en amont du conduit d'alimentation (12) d'un réservoir (8) destiné au stockage des réactifs et du solvant organique, et, en aval du conduit d'évacuation (15), d'un réservoir (16) pour le stockage après soutirage des produits bromés formés en cours de réaction, l'enceinte (2) étant reliée au niveau du décanteur inférieur (2b) à une pompe doseuse (11) destinée à pulser le milieu réactionnel.

27. Dispositif selon l'une des Revendications 24 à 26, **caractérisé en ce que** l'écoulement piston est assuré par une garde hydraulique (17) disposée immédiatement en sortie de colonne, en aval du conduit d'évacuation (15) et monté en série avec le réservoir (16).

28. Dispositif selon l'une des Revendications 25 à 27, **caractérisé en ce que** le réacteur est maintenu à une température comprise entre 0°C et la température ambiante.

29. Dispositif selon l'une des Revendications 25 à 27, **caractérisé en ce que** le réacteur est maintenu à une température comprise entre 0°C et 15°C.

## Patentansprüche

1. Verfahren zur Herstellung von 4'-Brommethyl-biphenyl Derivaten der allgemeinen Formel: 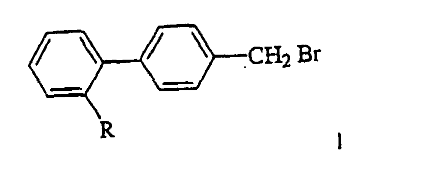 in der R:
• eine Cyanogruppe,
• eine Gruppe:
- CO₂R₁ oder - CONR₂R₃
in denen R₁, R₂ und R₃, die gleichartig oder verschieden sind, Wasserstoff oder eine geradkettige oder verzweigte C₁-C₆-Alkylgruppe, welche gegebenenfalls substituiert ist durch ein Halogenatom, eine Hydroxygruppe, Aminogruppe, C₁-C₄-Alkoxygruppe oder durch 1 bis 3 Phenylgruppen, die gegebenenfalls durch 1 bis 3 Gruppen ausgewählt aus Halogenatomen, Hydroxygruppen, Nitrogruppen, C₁-C₄-Alkylgruppen und C₁-C₄-Alkoxygruppen substituiert sind, darstellen,
• eine Tetrazolylgruppe der allgemeinen Formel: 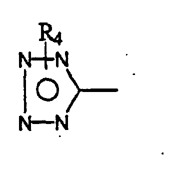
in der R₄ in der 1- oder 2-Stellung der Tetrazolylgruppe Wasserstoff oder eine Schutzgruppe darstellt, bedeutet, **dadurch gekennzeichnet, daß** man ein Biphenylderivat der allgemeinen Formel: 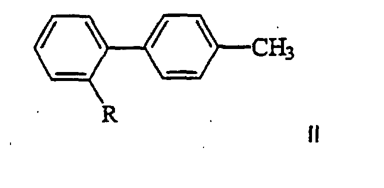 in der R die oben angegebenen Bedeutungen besitzt, in einem zweiphasigen Medium und unter der Wirkung einer Lichtbestrahlung, eines Bromierungsmittels, ausgewählt aus Brom, N-Bromacetamid, N-Bromphthalimid, N-Brommaleinimid, eines N-Bromsulfonamids, von N-Bromsuccinimid, 1,3-Dibrom-5,5-dimethylhydantoin und einem Bromwasserstoffsäure/Alkalimetallbromat-System zur Bildung der gewünschten Verbindung umsetzt.

2. Verfahren nach Anspruch 1, **dadurch gekennzeichnet, daß** R eine Tetrazolylgruppe darstellt, in der R₄ eine geradkettige oder verzweigte C₁-C₄-Alkylgruppe darstellt, die gegebenenfalls durch 1 bis 3 Phenylgruppen substituiert ist, die ihrerseits gegebenenfalls durch 1 bis 3 Gruppen ausgewählt aus Halogenatomen, Hydroxygruppen, Nitrogruppen, C₁-C₄-Alkylgruppen und C₁-C₄-Alkoxygruppen substituiert sind.

3. Verfahren nach Anspruch 1, **dadurch gekennzeichnet, daß** R eine Cyanogruppe, Tetrazolylgruppe, Triphenylmethyl-tetrazolylgruppe oder eine Gruppe - CO₂R₁ darstellt, worin R₁ Wasserstoff, eine Methyl-, Ethyl- oder Propyl-Gruppe bedeutet.

4. Verfahren nach einem der Ansprüche 1 oder 3, **dadurch gekennzeichnet, daß** R die Cyanogruppe bedeutet.

5. Verfahren nach einem der Ansprüche 1 bis 4, **dadurch gekennzeichnet, daß** das zweiphasige Medium aus Wasser und einem oder mehreren mit Wasser nicht mischbaren organischen Lösungsmittel(n) gebildet ist.

6. Verfahren nach Anspruch 5, **dadurch gekennzeichnet, daß** das organische Lösungsmittel ein halogenierter aliphatischer Kohlenwasserstoff oder halogenierter aromatischer Kohlenwasserstoff ist.

7. Verfahren nach Anspruch 5 oder 6, **dadurch gekennzeichnet, daß** das organische Lösungsmittel Dichlormethan ist.

8. Verfahren nach einem der Ansprüche 1 bis 7, **dadurch gekennzeichnet, daß** das zweiphasige Medium 0,1 bis 1 Volumen Wasser pro Volumen des organischen Lösungsmittels umfaßt.

9. Verfahren nach einem der Ansprüche 1 bis 8, **dadurch gekennzeichnet, daß** das zweiphasige Medium in einer Menge von 3 bis 30 Volumenäquivalenten pro Gewichtsäquivalent des Biphenylderivats der Formel II verwendet wird.

10. Verfahren nach einem der Ansprüche 1 bis 9, **dadurch gekennzeichnet, daß** als Bromierungsmittel Brom, N-Bromsuccinimid, 1,3-Dibrom-5,5-dimethylhydantoin oder ein Bromwasserstoffsäure/Alkalimetallbromat-System verwendet wird.

11. Verfahren nach einem der Ansprüche 1 bis 10, **dadurch gekennzeichnet, daß** das Bromierungsmittel in einer Menge von 0,5 bis 1,4 Moläquivalenten pro Moläquivalent des Biphenylderivats der Formel II verwendet wird.

12. Verfahren nach Anspruch 11, **dadurch gekennzeichnet, daß** das Bromierungsmittel in einer Menge von 0,95 bis 1,1 Moläquivalenten pro Moläquivalent des Biphenylderivats der Formel II verwendet wird.

13. Verfahren nach einem der Ansprüche 11 oder 12, **dadurch gekennzeichnet, daß** das Alkalimetallbromat in einer Menge von 0,3 bis 0,4 Moläquivalenten pro Moläquivalent des Biphenylderivats der Formel II verwendet wird.

14. Verfahren nach einem der Ansprüche 1 bis 13, **dadurch gekennzeichnet, daß** die Reaktion bei einer Temperatur zwischen 0°C und 45°C durchgeführt wird.

15. Verfahren nach Anspruch 14, **dadurch gekennzeichnet, daß** die Reaktion bei einer Temperatur zwischen 0°C und der Umgebungstemperatur durchgeführt wird.

16. Verfahren nach Anspruch 14 oder 15, **dadurch gekennzeichnet, daß** die Reaktion bei einer Temperatur zwischen 0°C und 15°C durchgeführt wird.

17. Verfahren nach einem der Ansprüche 1 bis 16, **dadurch gekennzeichnet, daß** die Lichtbestrahlung mit Hilfe einer Lichtquelle bewirkt wird, die Strahlung von 350 bis 600 nm emittiert.

18. Verfahren nach einem der Ansprüche 1 bis 14 und 17, **dadurch gekennzeichnet, daß** die Bromierungsreaktion kontinuierlich abläuft und die kontinulerliche Umwälzung der aus dem Bromierungsmittel und dem Biphenylderivat der Formel II gebildeten Reaktionsteilnehmer in einem Röhrenreaktor mit Kolbenausstoß durchgeführt wird, wobei der mit Licht bestrahlte Reaktor bei einer Temperatur zwischen 0°C und 45°C gehalten wird.

19. Verfahren nach Anspruch 18, **dadurch gekennzeichnet, daß** man die Bromierungsreaktion und die Extraktion der wasserlöslichen Nebenprodukte, die man anschließend aus dem Reaktionsmedium entfernt, gleichzeitig und in kontinuierlichen Phasen durchführt.

20. Verfahren nach Anspruch 19, **dadurch gekennzeichnet, daß** die Extraktions- und Entfernungsreaktion in einem Reaktor mit Kolbenausstoß durchgeführt werden, der aus einer Flüssig/Flüssig-Extraktionskolonne gebildet ist.

21. Verfahren nach einem der Ansprüche 18 bis 20, **dadurch gekennzeichnet, daß** es einerseits die Bromierungsreaktion in kontinuierlicher Phase durchgeführt wird durch Zirkulieren der durch das organische Lösungsmittel und die Reaktionsteilnehmer gebildeten Mischung in einem Röhrenreaktor mit Kolbenausstoß, der aus einer mit Licht bestrahlten und bei einer Temperatur zwischen 0°C und 45°C gehaltenen Flüssig/Flüssig-Extraktionskolonne gebildet ist, und andererseits die gleichzeitige und kontinuierliche Extraktion der wasserlöslichen Nebenprodukte welche durch das Wasser des zweiphasigen Mediums erfolgt, welches im Gegenstrom zu der Mischung in der Kolonne zirkuliert, gefolgt von der Entfernung der genannten wasserlöslichen Produkte aus dem Reaktionsmedium, umfaßt.

22. Verfahren nach einem der Ansprüche 18 bis 21, **dadurch gekennzeichnet, daß** die Temperatur zwischen 0°C und Umgebungstemperatur liegt.

23. Verfahren nach einem der Ansprüche 18 bis 22, **dadurch gekennzeichnet, daß** die Temperatur zwischen 0°C und 15°C liegt.

24. Photobromierungs-Vorrichtung zur Durchführung des Verfahrens gemäß einem der Ansprüche 18 bis 23 in kontinuierlicher Phase, **dadurch gekennzeichnet, daß** sie einen Röhrenreaktor mit Kolbenausstoß umfaßt, der aus einer mit Licht bestrahlten und bei einer Temperatur zwischen 0°C und 45°C gehaltenen Flüssig/Flüssig-Extraktionskolonne gebildet ist.

25. Vorrichtung nach Anspruch 24, umfassend (i) ein Reaktionsgefäß (1), gebildet aus einem ringförmigen Behälter (2) mit vertikaler Achse, der durch eine Innenwand (3) und in Abstand davon angeordnete Außenwand (4) gebildet ist und durch eine Scheibe (7), die im Bereich der unteren Dekantiereinrichtung (2b) des Behälters (2) angeordnet ist, und eine Scheibe (5), die im Bereich der oberen Dekantiereinrichtung (2a) des Behälters (2) angeordnet ist, verschlossen ist, (ii) eine photochemische Lampe (10), die mit einem doppelten Kühlmantel versehen und im Inneren des Behälters (9) angeordnet ist, der durch die Innenwand (3) des Behälters (2) und die Scheibe (5) begrenzt ist und die Scheibe (5) in Dickenrichtung durchdringt, (iii) eine Versorgungsleitung (12) zur Beschickung mit den Reaktionsteilnehmern und dem organischen Lösungsmittel, welche am Kopf der Kolonne angeordnet ist und die Scheibe (5) durchdringt und ihren Auslaß in der oberen Dekantiereinrichtung (2a) des Behälters (2) aufweist, (iv) eine Versorgungsleitung für Wasser (13), die am Fuß der Kolonne angeordnet ist und die Scheibe (7) durchdringt und ihren Auslaß in der unteren Dekantiereinrichtung (2b) des Behälters (2) aufweist, (v) eine Leitung (14) zum Abziehen der wässrigen Lösung, die am Kopf der Kolonne angeordnet ist und die Außenwand (4) des Behälters (2) im Bereich der oberen Dekantiereinrichtung (2a) durchdringt, und (vi) eine Leitung (15) zum Abziehen der Reaktionsprodukte am Fuß der Kolonne, welche die Scheibe (7) durchdringt, wobei der ringförmige Behälter (2) in seinem mittleren Bereich mit einem Wärmeaustauscher (6) ausgerüstet ist, der mit seiner Außenwand (4) in Kontakt steht, wobei die Innenwand (3) und die Außenwand (4) des Behälters (2) in ihrem mittleren Bereich mit Leitplatten (18) versehen sind.

26. Vorrichtung nach Anspruch 24 oder 25, **dadurch gekennzeichnet, daß** sie stromaufwärts der Versorgungsleitung (12) mit einem Vorratsbehälter (8) zur Lagerung der Reaktionsteilnehmer und des organischen Lösungsmittels und stromabwärts der Abziehleitung (15) mit einem Vorratsbehälter (16) zur Lagerung der im Verlaufe der Reaktion gebildeten bromierten Produkte nach dem Abziehen ausgerüstet ist, wobei der Behälter (2) im Bereich der unteren Dekantiereinrichtung (2b) mit einer Dosierpumpe (11) ausgerüstet ist, die dazu dient, das Reaktionsmedium in Pulsationen zu versetzen.

27. Vorrichtung nach einem der Ansprüche 24 bis 26, **dadurch gekennzeichnet, daß** Kolbenausstoß durch eine Flüssigkeitssperre (17) sichergestellt wird, die unmittelbar am Auslaß der Kolonne und stromabwärts der Abziehleitung (15) angeordnet ist und mit dem Vorratsbehälter (16) in Reihe geschaltet ist.

28. Vorrichtung nach einem der Ansprüche 25 bis 27, **dadurch gekennzeichnet, daß** das Reaktionsgefäß bei einer Temperatur zwischen 0°C und der Umgebungstemperatur gehalten wird.

29. Vorrichtung nach einem der Ansprüche 25 bis 27, **dadurch gekennzeichnet, daß** das Reaktionsgefäß bei einer Temperatur zwischen 0°C und 15°C gehalten wird.

## Claims

1. Process for preparing 4'-bromomethylbiphenyl derivatives of general formula: 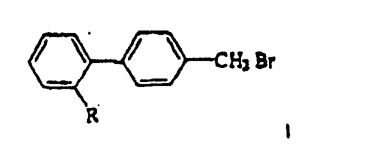 in which R represents:
• a cyano group
• a group:
-CO₂R₁ or -CONR₂R₃
in which R₁, R₂ and R₃, which may be identical or different, represent hydrogen or a linear or branched C₁-C₆ alkyl group which can be optionally substituted with a halogen atom, a hydroxyl, amino, C₁-C₄ alkoxy group or with 1 to 3 phenyl groups optionally substituted with 1 to 3 groups chosen from halogen atoms and hydroxyl, nitro, C₁-C₄ alkyl and C₁-C₄ alkoxy groups,
• a tetrazolyl group of general formula: 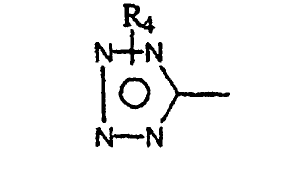
in which R₄, in position 1 or 2 of the tetrazolyl group, represents hydrogen or a protecting group, **characterized in that** a brominating agent chosen from bromine, N-bromoacetamide, N-bromophthalimide, N-bromomaleimide, an N-bromosulphonamide, N-bromosuccinimide, 1,3-dibromo-5,5-dimethylhydantoin and a hydrobromic acid/alkali metal bromate system is reacted, in a two-phase medium and under the effect of a photo-irradiation, with a biphenyl derivative of general formula: 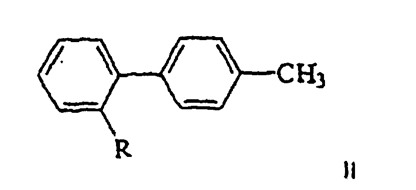 in which R has the same meaning as above, which gives the desired compound.

2. Process according to Claim 1, **characterized in that** R represents a tetrazolyl group in which R₄ represents a linear or branched C₁-C₆ alkyl group optionally substituted with 1 to 3 phenyl groups which are themselves optionally substituted with 1 to 3 groups chosen from halogen atoms and hydroxyl, nitro, C₁-C₄ alkyl and C₁-C₄ alkoxy groups.

3. Process according to Claim 1, **characterized in that** R represents a cyano, tetrazolyl or triphenylmethyltetrazolyl group or a group -CO₂R₁ in which R₁ represents hydrogen or a methyl, ethyl or propyl group.

4. Process according to one of Claims 1 or 3, **characterized in that** R represents a cyano group.

5. Process according to one of Claims 1 to 4, **characterized in that** the two-phase medium consists of water and of one or more water-immiscible organic solvents.

6. Process according to Claim 5, **characterized in that** the organic solvent is a halogenated aliphatic hydrocarbon or a halogenated aromatic hydrocarbon.

7. Process according to Claim 5 or 6, **characterized in that** the organic solvent is dichloromethane.

8. Process according to one of Claims 1 to 7, **characterized in that** the two-phase medium comprises from 0.1 to 1 volume of water per volume of organic solvent.

9. Process according to one of Claims 1 to 8, **characterized in that** the two-phase medium is used in a proportion of from 3 to 30 equivalents by volume per equivalent by weight of biphenyl derivative of formula II.

10. Process according to one of Claims 1 to 9, **characterized in that** the brominating agent is bromine, N-bromosuccinimide, 1,3-dibromo-5,5-dimethylhydantoin or a hydrobromic acid/alkali metal bromate system.

11. Process according to one of Claims 1 to 10, **characterized in that** the brominating agent is used in a proportion of from 0.5 to 1.4 molar equivalents per molar equivalent of biphenyl derivative of formula II.

12. Process according to Claim 11, **characterized in that** the brominating agent is used in a proportion of from 0.95 to 1.1 molar equivalents per molar equivalent of biphenyl derivative of formula II.

13. Process according to either of Claims 11 and 12, **characterized in that** the alkali metal bromate is used in a proportion of from 0.3 to 0.4 molar equivalent per molar equivalent of biphenyl derivative of formula II.

14. Process according to one of Claims 1 to 13, **characterized in that** the reaction is carried out at a temperature of between 0°C and 45°C.

15. Process according to Claim 14, **characterized in that** the reaction is carried out at a temperature of between 0°C and room temperature.

16. Process according to Claim 14 or 15, **characterized in that** the reaction is carried out at a temperature of between 0°C and 15°C.

17. Process according to one of Claims 1 to 16, **characterized in that** the photo-irradiation is obtained using a light source which emits radiation of 350 to 600 nm.

18. Process according to one of Claims 1 to 14 and 17, **characterized in that** the bromination reaction takes place in continuous phase and comprises the continuous circulation of the reagents consisting of the brominating agent and the biphenyl derivative of formula II in a tubular plug-flow reactor, this reactor being photo-irradiated and maintained at a temperature of between 0°C and 45°C.

19. Process according to Claim 18, **characterized in that** the bromination reaction and the extraction of the water-soluble by-products, which are then removed from the reaction medium, are carried out simultaneously and in continuous phases.

20. Process according to Claim 19, **characterized in that** the reaction, the extraction and the removal are carried out in a plug-flow reactor consisting of a liquid/liquid extraction column.

21. Process according to one of Claims 18 to 20, **characterized in that** it comprises, on the one hand, the continuous-phase bromination reaction by circulation of the mixture formed by the organic solvent and the reagents, in a tubular plug-flow reactor consisting of a liquid/liquid extraction column which is photo-irradiated and maintained at a temperature of between 0°C and 45°C, and, on the other hand, the simultaneous and continuous extraction of the water-soluble by-products, this being carried out using the water from the two-phase medium circulating counter-currentwise relative to the said mixture in the column, followed by the removal of the said water-soluble by-products from the reaction medium.

22. Process according to one of Claims 18 to 21, **characterized in that** the temperature is between 0°C and room temperature.

23. Process according to one of Claims 18 to 22, **characterized in that** the temperature is between 0°C and 15°C.

24. Photo-bromination device for carrying out the continuous-phase process according to one of Claims 18 to 23, **characterized in that** it comprises a tubular plug-flow reactor consisting of a liquid/liquid extraction column which is photo-irradiated and maintained at a temperature of between 0°C and 45°C.

25. Device according to Claim 24, comprising (i) a reactor (1) consisting of a circular chamber (2) with a vertical axis, delimited by an inner wall (3) and an outer wall (4) which are spaced apart, and closed off by a disc (7) located at the level of the bottom decanter (2b) of the chamber (2) and by a disc (5) located at the level of the top decanter (2a) of the chamber (2), (ii) a photochemical lamp (10) equipped with a cooling jacket, placed inside the chamber (9) delimited by the inner wall (3) of the chamber (2) and by the disc (5), passing through the said disc (5) in the direction of the thickness, (iii) a feed pipe (12) for loading the reagents and the organic solvent, located at the top of the column, passing through the disc (5) and emerging on the top decanter (2a) of the chamber (2), (iv) a water-feed pipe (13) located at the bottom of the column, passing through the disc (7) and emerging in the bottom decanter (2b) of the chamber (2), (v) a pipe (14) for discharging the aqueous solution, located at the top of the column, passing through the outer wall (4) of the chamber (2) at the level of the top decanter (2a), and (vi) a pipe (15) for discharging the reaction products, located at the bottom of the column, passing through the disc (7), the said circular chamber (2) being equipped in its central part with a heat exchanger (6) arranged in contact with its outer wall (4), the inner wall (3) and outer wall (4) of the chamber (2) being equipped in their central part with chicanes (18).

26. Device according to Claim 24 or 25, **characterized in that** it is equipped, upstream of the feed pipe (12), with a tank (8) intended for storing the reagents and the organic solvent, and, downstream of the discharge pipe (15), with a tank (16) for storage after removal of the brominated products formed during the reaction, the chamber (2) being connected at the level of the bottom decanter (2b) to a metering pump (11) intended to pulse the reaction medium.

27. Device according to one of Claims 24 to 26, **characterized in that** the plug flow is ensured by a hydraulic guard (17) placed immediately at the column outlet, downstream of the discharge pipe (15) and mounted in series with the tank (16).

28. Device according to one of Claims 25 to 27, **characterized in that** the reactor is maintained at a temperature of between 0°C and room temperature.

29. Device according to one of Claims 25 to 27, **characterized in that** the reactor is maintained at a temperature of between 0°C and 15°C.
